# EUROPÄISCHE PATENTANMELDUNG

(11) **EP 4 792 756 A1**
(43) Veröffentlichungstag der Anmeldung: **19.08.2026**
(21) Anmeldenummer: 26155908.2
(22) Anmeldetag: 03.02.2026
(51) Int. Cl.: A61B 17/15

(54) **OPERATIONSSCHABLONE UND VERFAHREN ZUR GESTALTUNG UND/ODER HERSTELLUNG EINER OPERATIONSSCHABLONE**

(30) Priorität: 14.02.2025 DE 102025105586
(71) Anmelder: Karl Leibinger Asset Management GmbH & Co. KG, 78570 Mühlheim an der Donau (DE)
(72) Erfinder: Aksu, Adem, 78054 VS-Schwenningen (DE); Reinauer, Frank, 78576 Emmingen-Liptingen (DE); Krist, Jens, 78567 Fridingen (DE); Wild, Heiner, 78589 Dürbheim (DE)
(74) Vertreter: Schwamberger, Martin

(57) **Zusammenfassung**

Die Erfindung betrifft eine Operationsschablone (OS) zur Anwendung im Bereich eines Schläfenbeins, insbesondere im Rahmen einer Durchführung einer radikalen Mastoidektomie, umfassend einen Schablonenkörper (SK) mit einer Außenseite (AS) und einer Innenseite (IS), auf welcher der Schablonenkörper (SK) schläfenbeinseitig aufzulegen ist. Der Schablonenkörper (SK) ist mit einem von der Außenseite (AS) zu der Innenseite (IS) verlaufenden Durchbruch (D) durchsetzt, wobei der Durchbruch (D) bei dem Schablonenkörper (SK) mit einem Außenrand (AR) definiert ist, welcher einer Außenkontur eines zu entfernenden Knochenteils des Schläfenbeins (SB) entspricht.

## Beschreibung

Die Erfindung betrifft eine Operationsschablone zur Anwendung im Bereich eines Schläfenbeins, insbesondere im Rahmen einer Durchführung einer radikalen Mastoidektomie, umfassend einen Schablonenkörper mit einer Außenseite und einer Innenseite, auf welcher der Schablonenkörper schläfenbeinseitig aufzulegen ist. Des Weiteren betrifft die Erfindung ein Verfahren zur Gestaltung und/oder Herstellung einer Operationsschablone für eine Anwendung im Bereich eines Schläfenbeins.

Bei chronischen Erkrankungen im Bereich des Mittelohres kann in bestimmten Fällen eine operative Behandlung in Form einer radikalen Mastoidektomie erforderlich sein, bei welcher bei einem Schläfenbein (Os temporale) ein Ausräumen eines Warzenteils (Pars mastoidea) unter Entfernung der hinteren Gehörgangswand vorgenommen wird, um eine gemeinsame Höhle aus einem Kuppelraum des Mittelohres, dem Warzenteil und dem Gehörgang zu bilden. Diese Höhle wird dabei häufig auch als Mastoidhöhle oder Radikalhöhle bezeichnet. So kann eine radikale Mastoidektomie häufig im Falle eines ausgeprägten Cholesteatoms notwendig sein, bei welchem es sich um eine chronische Knocheneiterung des Mittelohres handelt. Ziel der radikalen Mastoidektomie ist eine vollständige Entfernung einer Matrix des Cholesteatoms. Bei im Bereich des Schädels und hierbei auch des Schläfenbeins durchzuführenden Operationen ist zudem allgemein die Verwendung von Operationsschablonen bekannt, um den Chirurgen bei der Durchführung der jeweiligen Operation zu unterstützen.

So geht aus der CN 215079279 U eine Operationsschablone in Form einer Kraniotomie-Schablone hervor. Diese Kraniotomie-Schablone weist einen Schablonenkörper auf, der dafür vorgesehen ist, auf einer Innenseite auf einem Schläfenbein aufgelegt zu werden. An einer der Innenseite abgewandt liegenden Außenseite ist auf dem Schablonenkörper ferner ein Anlagebereich für eine Ohrmuschel eines Patienten ausgestaltet, um die Kraniotomie-Schablone beim Auflegen auf dem Schläfenbein des Patienten korrekt positionieren zu können. Außerdem ist in dem Schablonenkörper ein Sichtfenster sowie angrenzend hierzu eine Nadelführung ausgestaltet.

Ausgehend vom vorstehend beschriebenen Stand der Technik ist es nun die Aufgabe der vorliegenden Erfindung eine Operationsschablone zu schaffen, mittels welcher ein Chirurg bei der Durchführung einer Operation im Bereich des Schläfenbeins, bevorzugt bei der Durchführung einer radikalen Mastoidektomie, unterstützt werden kann.

Aus vorrichtungstechnischer Sicht erfolgt eine Lösung dieser Aufgabe ausgehend vom Oberbegriff des nebengeordneten Anspruchs 1 in Verbindung mit dessen kennzeichnenden Merkmalen. Zudem erfolgt eine Lösung der Aufgabe aus verfahrenstechnischer Sicht durch die technischen Merkmale des nebengeordneten Anspruchs 17. Die hierauf jeweils folgenden, abhängigen Ansprüche geben jeweils vorteilhafte Weiterbildungen der Erfindung wieder. Weitere vorteilhafte Ausgestaltungen ergeben sich aus der Beschreibung sowie aus den Figuren.

Gemäß dem nebengeordneten Anspruch 1 umfasst eine Operationsschablone, welche zur Anwendung im Bereich eines Schläfenbeins vorgesehen ist, einen Schablonenkörper mit einer Außenseite und einer Innenseite, auf welcher der Schablonenkörper schläfenbeinseitig aufzulegen ist. Bevorzugt ist die Operationsschablone dabei für die Verwendung im Rahmen einer radikalen Mastoidektomie vorgesehen, durch welche operativ eine Mastoidhöhle bzw. Radikalhöhle gebildet wird, beispielsweise um ein Cholesteatom zu behandeln.

Die erfindungsgemäße Operationsschablone weist dabei einen Schablonenkörper auf, welcher für eine Auflage an einem Schläfenbein (Os temporale) gestaltet ist. Bei Verwendung der Operationsschablone ist dieser Schablonenkörper dabei mit seiner Innenseite auf dem Schläfenbein aufzulegen, wobei dies im Vorfeld der am Schläfenbein vorzunehmenden Operation, also insbesondere vor Durchführung der radikalen Mastoidektomie, vorzunehmen ist.

Im Rahmen der Erfindung ist der Schablonenkörper vorzugsweise im Rahmen eines additiven Herstellungsverfahrens ausgestaltet worden, wobei dies bevorzugt mittels eines 3D-Druckverfahrens stattgefunden hat.

Bevorzugt besteht der Schablonenkörper aus einem Kunststoff, bei welchem es sich insbesondere um ein Polymer und hierbei besonders bevorzugt um einen Thermoplast handelt, wie beispielsweise Polyamid (PA) oder Polyetheretherketon (PEEK). Alternativ dazu kann der Schablonenkörper auch aus einem metallischen Werkstoff, wie insbesondere Titan oder Edelstahl, oder einer Metalllegierung, bevorzugt einer Titanlegierung, bestehen. Darüber hinaus könnte der Schablonenkörper auch aus unterschiedlichen Werkstoffen bestehen, um lokal unterschiedliche Eigenschaften zu realisieren, beispielsweise lokal unterschiedliche Steifigkeiten. Der Schablonenkörper kann einteilig oder auch mehrteilig ausgebildet sein.

Entsprechend dem nebengeordneten Anspruch 1 umfasst die Erfindung nun die technische Lehre, dass der Schablonenkörper mit einem von der Außenseite zu der Innenseite verlaufenden Durchbruch durchsetzt ist. Dabei ist der Durchbruch bei dem Schablonenkörper mit einem Außenrand definiert, welcher einer Außenkontur eines zu entfernenden Knochenteils des Schläfenbeins entspricht. Mit anderen Worten ist der Schablonenkörper also mit einem Durchbruch ausgestattet, welcher von der Außenseite des Schablonenkörpers zu der Innenseite des Schablonenkörpers verläuft und den Schablonenkörper vollständig durchdringt. Dieser Durchbruch ist dabei mit einem Außenrand im Schablonenkörper ausgestaltet, wobei der Außenrand identisch zu einer Außenkontur ist, welche ein bei dem Schläfenbein zu entfernender Knochenteil aufweist.

Eine derartig gestaltete Operationsschablone hat dabei den Vorteil, dass somit einem Chirurgen über den Durchbruch genau kenntlich gemacht werden kann, welcher Knochenteil im Bereich des Schläfenbeins zu entfernen ist. Dabei wird über den Außenrand des Durchbruchs zudem eine Führung realisiert, entlang welcher der die Operation durchführende Chirurg mit einem Operationswerkzeug, insbesondere einer Knochensäge, entlangfahren kann. Hierdurch ist eine genaue Entfernung des Knochenteils möglich. Insgesamt kann hierdurch eine im Bereich des Schläfenbeins durchzuführende Operation sicherer gestaltet werden. Insbesondere lässt sich hierdurch eine radikale Mastoidektomie auf zuverlässige Weise durchführen.

Der Durchbruch weist bei der Operationsschablone vorzugsweise einen geschlossenen Außenrand auf, indem der Durchbruch inselähnlich vollständig von Material des Schablonenkörpers umgeben ist. Bevorzugt bildet der Durchbruch eine Sägeschablone, indem durch den Außenrand des Durchbruchs die Außenkontur eines durch Sägen zu entfernenden Knochenteils definiert ist. Bei Anwendung wird über den Durchbruch dabei insbesondere eine Außenkontur eines zu entfernenden Knochenteils eines Warzenteils (Pars mastoidea) und/oder eines Paukenteils (Pars tympanica) des Schläfenbeins definiert.

Entsprechend einer möglichen Ausführungsform der Erfindung ist der Außenrand zumindest abschnittsweise durch eine Innenkontur eines Führungsabschnitts gebildet. Durch das Vorsehen des Führungsabschnitts, welcher mit seiner Innenkontur den Außenrand des Durchbruchs definiert, wird das Entlangführen eines Operationswerkzeugs beim Entfernen des Knochenteils vereinfacht. Bevorzugt ist der Führungsabschnitt dabei gegenüber dem restlichen Schablonenkörper zu der Außenseite hin vorstehend ausgestaltet, um eine bessere Führung zu gewährleisten.

Gemäß einer weiteren Ausgestaltungsmöglichkeit der Erfindung ist der Schablonenkörper mit einem Befestigungsabschnitt versehen, an welchem von der Außenseite zu der Innenseite verlaufende Durchgangsöffnungen ausgestaltet sind. Dabei dienen diese Durchgangsöffnungen zur Hindurchführung je eines Befestigungsmittels für eine schläfenbeinseitige Befestigung der Operationsschablone, wobei diese Befestigung bevorzugt an einen Warzenteil des Schläfenbeins erfolgt. Dies hat den Vorteil, dass durch die Befestigung ein Verrutschen der Operationsschablone bei Durchführung der Operation zuverlässig verhindert werden kann. Insbesondere können dabei durch die gebildeten Durchgangsöffnungen Knochenschrauben hindurchgeführt werden.

Bei Kombination der beiden vorstehend beschriebenen Varianten umgibt der Befestigungsabschnitt den Führungsabschnitt zumindest abschnittsweise peripher, wobei der Führungsabschnitt bei dem Schablonenkörper gegenüber dem Befestigungsabschnitt zu der Außenseite hin vorstehend ausgestaltet ist. Hierdurch kann eine umliegende Befestigung der Operationsschablone zu dem zu entfernenden Knochenteil realisiert werden. Insbesondere weist der Befestigungsabschnitt dabei im Vergleich zu dem Führungsabschnitt eine geringere Erstreckung zu der Außenseite hin auf.

Alternativ dazu kann der Führungsabschnitt stufenlos in den Befestigungsabschnitt übergehen, sodass der Führungsabschnitt keine erhöhte Führung für das Sägeblatt bildet. Dadurch kann die Operationsschablone kompakter ausgestaltet werden. Eine erhöhte Führung für das Sägeblatt ist in zahlreichen Fällen aufgrund der geringen Wandstärke des Schläfenbeins nicht erforderlich.

Entsprechend einer Ausführungsform der Erfindung weist der Schablonenkörper einen Abdeckabschnitt auf, welcher eine Trennwand bildet. Die Trennwand teilt dabei im Bereich des Abdeckabschnitts die Außenseite und die Innenseite voneinander ab und ist für ein zumindest überwiegendes Überdecken eines Gehörgangs durch die Operationsschablone vorgesehen. Dadurch kann mithilfe der Operationsschablone ein unbeabsichtigtes Verletzen des Gehörgangs bei Durchführung der Operation wirksam verhindert werden. Denn bei Anordnung der Operationsschablone wird der Gehörgang, insbesondere der Gehörkanal des Mittelohrs, über den Abdeckabschnitt der Operationsschablone verdeckt, wodurch ein Abrutschen in den Gehörgang oder auch ein sonstiges, unabsichtliches Verletzen des Gehörgangs mit einem OP-Werkzeug ausgeschlossen ist. Besonders bevorzugt ist der Abdeckabschnitt dabei dazu ausgestaltet, den gesamten Gehörgang abzudecken. Insbesondere ist der Abdeckabschnitt kappenartig gestaltet, d.h. der Abdeckabschnitt weist bevorzugt eine an eine Schutzkappe orientierte Gestalt auf. Bevorzugt bildet die Trennwand zudem mit einer Kante einen Abschnitt des Außenrandes des Durchbruchs, so dass der Abdeckabschnitt bei dem Schablonenkörper dann angrenzend zu dem Durchbruch ausgebildet. Hierdurch verhindert der Abdeckabschnitt mit der Trennwand wirksam das unbeabsichtigte Verletzen des Gehörgangs, während mit einem OP-Werkzeug in dem Durchbruch zum Entfernen des Knochenteils gearbeitet wird.

In Weiterbildung der vorgenannten Ausführungsform ist der Abdeckabschnitt zu der Innenseite hin gewölbt gestaltet und dient einer Einführung in den Gehörgang. Dabei bildet der Abdeckabschnitt neben der Trennwand zudem eine Seitenwand, die gegenüber der Trennwand zu der Außenseite hin vorsteht. In vorteilhafter Weise kann die Operationsschablone dadurch mit dem Abdeckabschnitt in den Gehörgang eingeführt und damit ein besonders zuverlässiger Schutz des Gehörgangs erreicht werden. Zudem wird über die Seitenwand auch ein seitlicher Schutz realisiert. Insbesondere ist der gewölbte Abdeckabschnitt dabei schaufelartig gewölbt gestaltet. Die bei dem Abdeckabschnitt definierte Innenwand kann für ein zumindest abschnittsweises Anlegen an eine Innenwand des Gehörgangs vorgesehen und/oder gestaltet sein. Bevorzugt umgibt die Seitenwand die Trennwand bei dem Abdeckabschnitt zumindest abschnittsweise peripher.

Weiter alternativ oder ergänzend ist der Abdeckabschnitt bei dem Schablonenkörper an einem Ende vorgesehen, mit welchem der Schablonenkörper zu der Innenseite hin verkippt ist. Der Schablonenkörper ist in diesem Fall also abschnittweise, nämlich mit dem Ende, zu der Innenseite hin verkippt. Dabei definiert der Abdeckabschnitt einen Abschnitt des Außenrandes des Durchbruchs durch eine Kante der Trennwand. Vorzugsweise bildet der Abdeckabschnitt an der Trennwand der Außenseite zugewandt eine Auflage, welche einem Auflegen eines zum Entfernen des Knochenteils des Schläfenbeins zu verwendenden Operationswerkzeugs dient und eine Resektionstiefe definiert. Dies hat den Vorteil, dass somit über den Abdeckabschnitt der Operationsschablone zusätzlich auch eine Referenz für die Tiefe des zu entfernenden Knochens gebildet wird. So kann der die Operation durchführende Chirurg das Instrument auf dem Abdeckabschnitt auflegen und hiervon ausgehend die Resektion des Knochenteils vornehmen. Das Verkippen des Endes ist dabei insbesondere hinsichtlich eines Befestigungsabschnitts vorgenommen, welcher für eine schläfenbeinseitige Befestigung der Operationsschablone vorgesehen ist.

Bei Kombination der vorstehenden Variante mit der Ausgestaltungsmöglichkeit der Erfindung, nach welcher der Außenrand des Durchbruchs zumindest abschnittsweise durch eine Innenkontur des Führungsabschnitts gebildet ist, ist diese Bildung des Außenrandes des Durchbruchs besonders bevorzugt abseits des verkippten Endes vorgenommen. In der Folge kann der Chirurg im Rahmen der Operation seitlich ausgehend von der Auflage des Abdeckabschnitts in den zu entfernenden Knochen hineinarbeiten.

Es ist eine weitere mögliche Ausführungsform der Erfindung, dass an dem Schablonenkörper zudem mindestens ein Bohrführungsabschnitt ausgebildet ist, welcher zumindest einen von der Außenseite zu der Innenseite verlaufenden Bohrkanal bildet und der jeweils einer Führung eines Knochenbohrers zum Entfernen je eines Knochenteils des Schläfenbeins dient. Hierdurch kann die Operationsschablone einen Chirurgen auch bei einer Entfernung eines Knochenteil des Schläfenbeins mittels Bohren unterstützen, wodurch insbesondere in Tiefenrichtung verlaufende Teile des Schläfenbeins zuverlässig entfernt werden können. So handelt es sich bei dem über den Knochenbohrer zu entfernenden Knochenteil bevorzugt um einen Teil eines Paukenteils (Pars tympanica) des Schläfenbeins.

In Weiterbildung der vorgenannten Ausführungsform kann der mindestens eine Bohrführungsabschnitt zumindest an einem an der Außenseite liegenden Ende mit jeweils einem Führungsbereich versehen sein, in welchem ein Innendurchmesser des Bohrkanals an einen Außendurchmesser des Knochenbohrers angenähert ist. Hierdurch wird für den durchführenden Chirurgen eine geeignete Führung des Knochenbohrers an der Operationsschablone verwirklicht. Besonders bevorzugt ist der jeweils eine Führungsbereich durch eine Führungshülse gebildet, welche an dem mindestens einen Bohrführungsabschnitt in den Schablonenkörper eingebracht ist. Letzteres ist insbesondere dann verwirklicht, wenn der Schablonenkörper aus einem Polymer hergestellt ist, wobei die eingebrachte Führungshülse dann aus einem metallischen Werkstoff gebildet ist. In vorteilhafter Weise kann hierdurch im Bereich des jeweiligen Bohrführungsabschnitts eine höhere Festigkeit dargestellt werden.

Alternativ oder auch ergänzend zu der vorgenannten Weiterbildung ist an dem mindestens einen Bohrführungsabschnitt bündig an einer außenseitigen Mündung des Bohrkanals eine Anschlagsfläche ausgestaltet, welche einen Anschlag für ein den Knochenbohrer führendes Operationswerkzeug bildet. Eine Länge des jeweiligen Bohrkanals ist dabei entsprechend einer Bohrtiefe gestaltet, welche über den Knochenbohrer auf der Innenseite bei Anschlag des Operationswerkzeug an der jeweils einen Anschlagsfläche darzustellen ist. Dies hat den Vorteil, dass durch den gebildeten Anschlag eine eindeutige Bohrtiefe definiert und dadurch auch ein zu tiefes Bohren zuverlässig ausgeschlossen werden kann.

Besonders bevorzugt sind an der Operationsschablone mehrere Bohrführungsabschnitte ausgestaltet. In der Folge definiert die Operationsschablone somit mehrere über einen Knochenbohrer zu entfernende Knochenteile.

Bei Kombination der Ausgestaltung des Führungsabschnitts mit der Ausgestaltung des mindestens einen Bohrführungsabschnitts ist der mindestens eine Bohrführungsabschnitt an dem Führungsabschnitt angrenzend zu der Innenkontur ausgestaltet. Dadurch wird über den durch den Führungsabschnitt umrandeten Durchbruch ein zu entfernender Knochenteil sowie über den mindestens einen Bohrführungsabschnitt ein weiterer zu entfernender, hierzu angrenzender Knochenteil definiert.

Entsprechend einer Ausgestaltungsmöglichkeit der Erfindung ist der Schablonenkörper patientenspezifisch ausgeführt. In vorteilhafter Weise ist die Operationsschablone somit speziell auf den jeweiligen Patienten zugeschnitten. Insbesondere ist dabei zumindest der Verlauf des Außenrandes des Durchbruchs, die Größe des Durchbruchs, die Lage des Durchbruchs am Schablonenkörper sowie ggf. die Lage des mindestens einen Bohrführungsabschnitts bei der Operationsschablone an den jeweiligen Patienten angepasst. Alternativ dazu kann der Schablonenkörper nicht-patientenspezifisch geformt sein. In diesem Fall weist der Schablonenkörper eine vorab definierte Standard-Form aufweist, welche einer üblichen menschlichen Anatomie entspricht. Der Schablonenkörper kann dazu in verschiedenen Größen bereitgestellt werden.

In Weiterbildung der vorgenannten Ausgestaltungsmöglichkeit kann der Schablonenkörper auf der Innenseite mit einer Auflagekontur ausgeführt sein, welche zumindest annähernd als Gegenstück zu einer Oberflächenkontur des Schläfenbeins im aufzulegenden Bereich gestaltet ist. Hierdurch kann die schläfenbeinseitige Auflage des Schablonenkörpers auf geeignete Weise erfolgen.

Insbesondere ist der Bereich, in welchem die Operationsschablone auf das Schläfenbein aufzulegen ist, für die patientenspezifische Ausführung der Operationsschablone im Vorfeld im Rahmen eines medizinischen, bildgebenden Verfahrens erfasst worden, wie beispielsweise mittels CT.

Gemäß dem nebengeordneten Anspruch 13 betrifft die Erfindung zudem ein Verfahren zur Gestaltung und/oder Herstellung einer Operationsschablone für eine Anwendung im Bereich eines Schläfenbeins. Dabei wird ein Schablonenkörper mit einer Außenseite und einer Innenseite gestaltet, auf welcher der Schablonenkörper schläfenbeinseitig aufzulegen ist. Bei dem Schablonenkörper wird ein von der Außenseite zu der Innenseite verlaufender Durchbruch vorgesehen, wobei bei dem Durchbruch ein Außenrand definiert wird, welcher einer Außenkontur eines zu entfernenden Knochenteils des Schläfenbeins entspricht.

Im Rahmen des erfindungsgemäßen Verfahrens wird also eine Gestaltung oder eine Herstellung oder sowohl eine Gestaltung, als auch eine Herstellung einer Operationsschablone durchgeführt. Dabei ist unter einer "Gestaltung" insbesondere eine Planung bzw. Konstruktion der Operationsschablone zu verstehen, also eine Erstellung eines Entwurfs bzw. einer Vorlage für eine Herstellung der Operationsschablone. Mit "Herstellung" ist eine Fertigung der Operationsschablone gemeint, also deren physische Ausgestaltung. Insbesondere findet im Rahmen des erfindungsgemäßen Verfahrens sowohl die Gestaltung, als auch die Herstellung einer Operationsschablone statt. Eine Herstellung wird bevorzugt im Rahmen eines additiven Herstellungsverfahrens vorgenommen, besonders bevorzugt im Rahmen eines 3D-Druckverfahrens.

In vorteilhafter Weise kann hierdurch eine Operationsschablone gestaltet und/oder hergestellt werden, bei deren Verwendung einem Chirurgen genau kenntlich gemacht ist, welcher Knochenteil im Bereich des Schläfenbeins zu entfernen ist. Dabei kann über den Außenrand des Durchbruchs zudem eine Führung dargestellt werden, entlang welcher der die Operation durchführende Chirurg mit einem Operationswerkzeug, insbesondere einer Knochensäge, entlangfahren kann. Hierdurch ist eine genaue Entfernung des Knochenteils möglich. Insgesamt kann hierdurch eine im Bereich des Schläfenbeins durchzuführende Operation sicher gestaltet werden. Bevorzugt kann ein Chirurg dabei bei Durchführung einer radikalen Mastoidektomie unterstützt werden.

Bevorzugt wird die Operationsschablone bei der Gestaltung und/oder bei der Erstellung zudem entsprechend wenigstens einer der vorstehend beschriebenen Varianten einer Operationsschablone ausgestaltet.

Es ist eine weitere Ausgestaltungsmöglichkeit des erfindungsgemäßen Verfahrens, dass gemeinsam mit der Operationsschablone zudem ein Schläfenbeinimplantat geplant und/oder hergestellt wird. In vorteilhafter Weise kann dieses Schläfenbeinimplantat dann nach Durchführung der radikalen Mastoidektomie verwendet werden, um eine durch Entfernung des Knochenteils gebildete Fehlstelle des Schläfenbeins zu überdecken.

Eine vorteilhafte Ausführungsform der Erfindung, die nachfolgend erläutert wird, ist in den Zeichnungen dargestellt. Es zeigen:
- Fig. 1: eine perspektivische Darstellung einer Operationsschablone entsprechend einer Ausführungsform;
- Fig. 2: eine perspektivische Ansicht eines Teils eines Schädels im Bereich eines Schläfenbeins mit einem markierten, zu entfernenden Bereich des Schläfenbeins;
- Fig. 3 bis 6: weitere perspektivische Darstellungen des Bereichs des Schädels, nach einem Aufsetzen der Operationsschablone aus Fig. 1;
- Fig. 7: eine weitere perspektivische Darstellung des Bereichs des Schädels, nach einem Entfernen des Bereichs des Schläfenbeins;
- Fig. 8: eine weitere Ansicht des Schädels nach einem Einsetzen eines Schläfenbeinimplantats;
- Fig. 9 bis 11: unterschiedliche Ansichten einer Operationsschablone gemäß einer weiteren Ausgestaltungsmöglichkeit der Erfindung; und
- Fig. 12 und 13: Darstellungen eines Bereichs eines Schädels nach einem Aufsetzen der Operationsschablone aus den Fig. 9 bis 11.

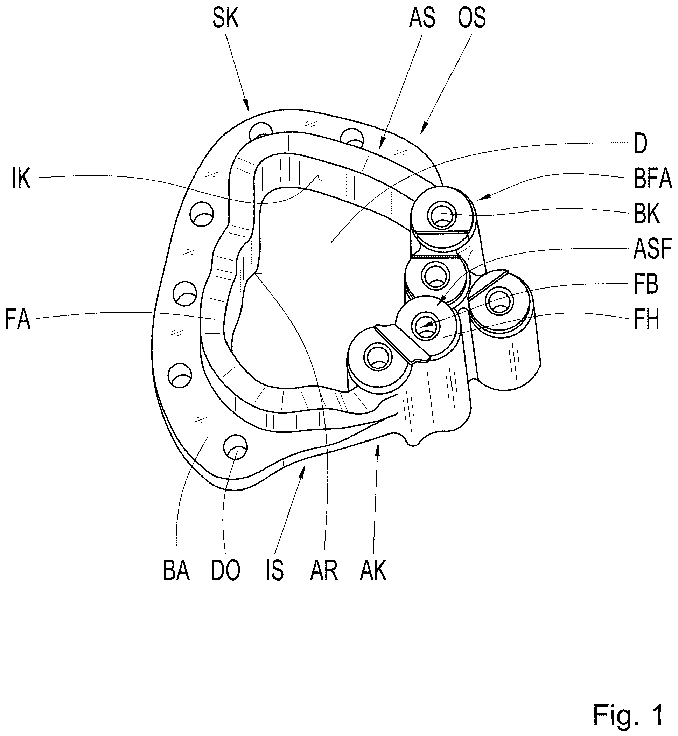

Aus Fig. 1 geht eine perspektivische Darstellung einer Operationsschablone OS hervor, welche entsprechend einer Ausführungsform der Erfindung ausgebildet ist. Die Operationsschablone OS weist einen Schablonenkörper SK auf, welcher an einer Innenseite IS für eine Auflage auf einem Schläfenbein gestaltet ist. Der Schablonenkörper SK ist mit einem Durchbruch D durchsetzt, indem der Durchbruch D bei dem Schablonenkörper SK von der Innenseite IS zu einer gegenüberliegend zur Innenseite IS angeordneten Außenseite AS verläuft.

Der Durchbruch D weist einen Außenrand AR auf, der bei der Operationsschablone OS durch eine Innenkontur IK eines Führungsabschnitts FA des Schablonenkörpers SK definiert ist. Dabei ist der Durchbruch D inselartig an dem Schablonenkörper SK ausgestaltet, indem der Führungsabschnitt FA den Durchbruch D vollständig umgrenzt und der Außenrand AR geschlossen durch die Innenkontur IK definiert wird.

Außen ist der Führungsabschnitt FA abschnittsweise peripher vor einem Befestigungsabschnitt BA umgeben, der mit mehreren Durchgangsöffnungen DO versehen ist, wobei die Durchgangsöffnungen DO dabei jeweils von der Innenseite IS zu der Außenseite AS verlaufen. Der Führungsabschnitt FA ist gegenüber dem Befestigungsabschnitt BA zu der Außenseite AS des Schablonenkörpers SK vorstehend ausgebildet.

An dem Führungsabschnitt FA sind zudem angrenzend zu dem Durchgang D mehrere Bohrführungsabschnitte BFA ausgestaltet, die jeweils einen von der Außenseite AS zu der Innenseite IS durchgehend verlaufenden Bohrkanal BK bilden. Die Bohrführungsabschnitte BFA sind dabei teilweise mit unterschiedlichen Erstreckungen zwischen der Innenseite IS und der Außenseite AS ausgestaltet, wodurch sich unterschiedliche Längen der Bohrkanäle BK ergeben.

Der Schablonenkörper SK der Operationsschablone OS ist im Rahmen eines additiven Herstellungsverfahrens aus einem Polymer hergestellt worden, wobei die Herstellung dabei bevorzugt im Rahmen eines 3D-Druckverfahrens stattgefunden hat. Als Polymer ist dabei insbesondere ein Thermoplast verwendet worden, wie beispielsweise Polyethylen (PE) oder Polyetheretherketon (PEEK). An den außenseitigen Mündungen der Bohrführungsabschnitte BFA sind Führungshülsen FH in den Schablonenkörper SK eingebracht, wobei diese Führungshülsen FH dabei insbesondere aus einem metallischen Werkstoff, beispielsweise Titan, bestehen.

Die Führungshülsen FH bilden bei dem jeweils zugehörigen Bohrführungsabschnitt BFA einen Führungsbereich FB, in welchem ein Innendurchmesser des Bohrkanal BK über die Länge der jeweiligen Führungshülse FH verkleinert ist. Außerdem wird über die Führungshülsen FH auf der Außenseite AS je eine Anschlagsfläche ASF definiert, die dabei an der jeweiligen Mündung des jeweiligen Bohrkanal BK bündig ausgestaltet ist.

Zudem ist der Schablonenkörper SK auf der Innenseite IS mit einer - in Fig. 1 nur teilweise zu erkennenden - Auflagekontur AK versehen.

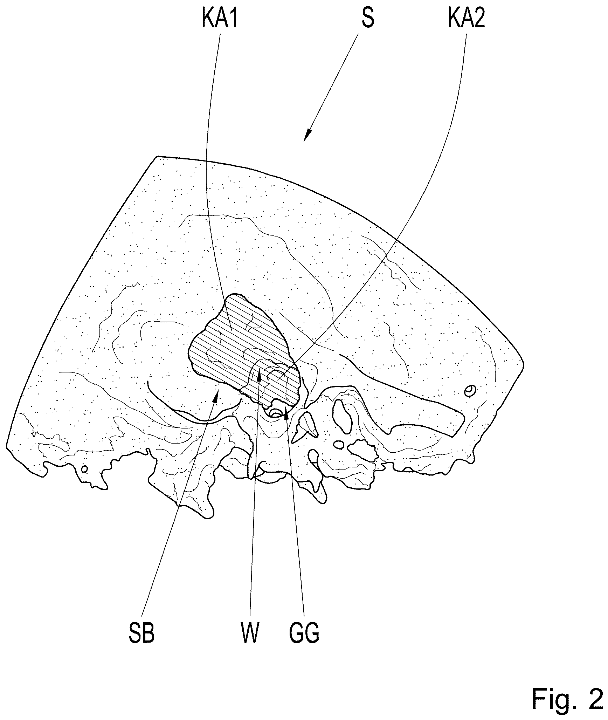

Die in Fig. 1 dargestellte Operationsschablone OS ist für die konkrete, patientenspezifische Anwendung im Bereich eines Schläfenbeins SB eines Schädels S gestaltet und hergestellt worden. Dazu wurde die Gestalt dieses Schädels S im Vorfeld mittels eines medizinischen, bildgebenden Verfahrens, insbesondere mittels CT, unter Berücksichtigung eines in den Fig. 2 bis 8 jeweils teilweise dargestellten 3D-Modells des Schädels S ermittelt. Hierbei ist die Operationsschablone OS zum einen dadurch patientenspezifisch ausgestaltet, dass der Durchbruch D und die Bohrführungsabschnitte BFA an der Operationsschablone OS in Abhängigkeit von Knochenabschnitten KA1 und KA2 gestaltet sind, welche bei dem Schläfenbein SB des Schädels S als im Rahmen einer radikalen Mastoidektomie zu entfernende Bereiche deklariert worden und in Fig. 2 schraffiert angedeutet sind. Die radikale Mastoidektomie ist dabei insbesondere dazu durchzuführen, um ein Cholesteatom im Bereich eines äußeren Gehörgangs GG vollständig entfernen zu können. Zum anderen ist die Auflagekontur AK des Schablonenkörpers SK zumindest annähernd als Gegenstück zu einer Oberflächenkontur des Schläfenbeins SB im umgebenden Bereich der Knochenabschnitte KA1 und KA2 gestaltet.

Vorliegend handelt es sich bei dem zu entfernenden Knochenabschnitt KA1 um einen Abschnitt eines Warzenteils (Pars mastoidea) des Schläfenbeins SB, während der zu entfernenden Knochenabschnitt KA2 ein Abschnitt eines Paukenteils (Pars tympanica) des Schläfenbeins SB ist und hierbei einen Teil der hinteren, knöchernen Wand W des äußeren Gehörganges GG bildet.

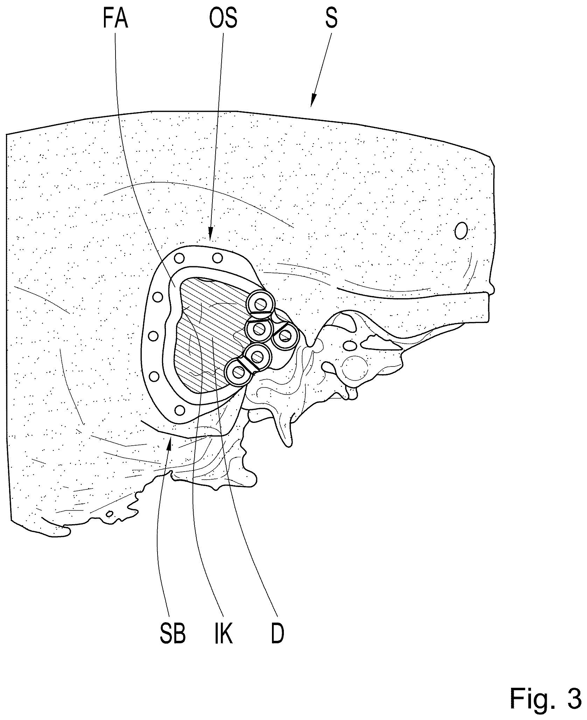

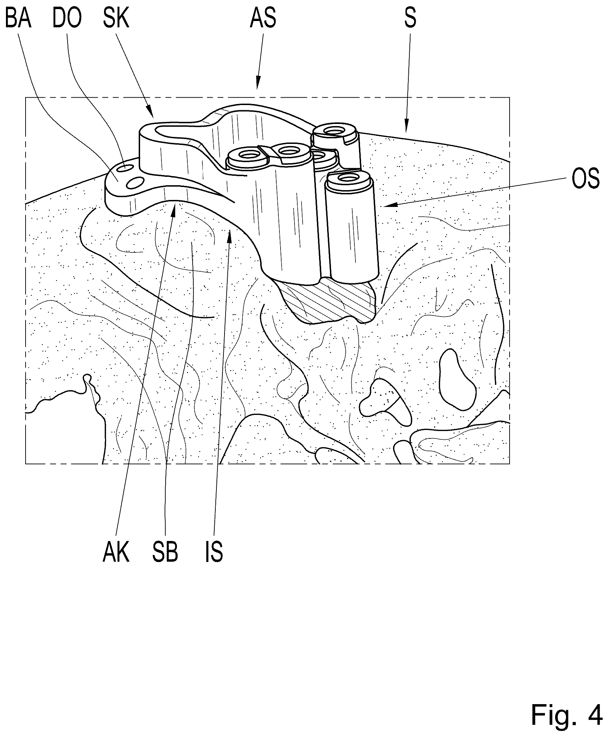

Fig. 3 bis Fig. 6 zeigen die Operationsschablone OS im auf das Schläfenbein SB aufgesetzten Zustand, wobei dabei insbesondere aus Fig. 4 die Auflage des Schablonenkörpers SK mit der an der Innenseite IS ausgestalteten Auflagekontur AK auf dem Schläfenbein SB zu erkennen ist. Die Operationsschablone OS wird über den Befestigungsabschnitt BA am Schläfenbein SB befestigt, wobei hierzu insbesondere Knochenschrauben durch die Durchgangsöffnungen DO zum Verschrauben der Operationsschablone OS mit dem Schläfenbein SB hindurchgeführt werden.

Über den Durchgang D der Operationsschablone OS wird einem operierenden Chirurgen der mittels einer Knochensäge zu entfernende Bereich der Knochenabschnitte KA1 und KA2 aufgezeigt, wobei der Chirurg hierbei beim Arbeiten mit der Knochensäge an der Innenkontur IK des Führungsabschnitts FA entlangfahren kann.

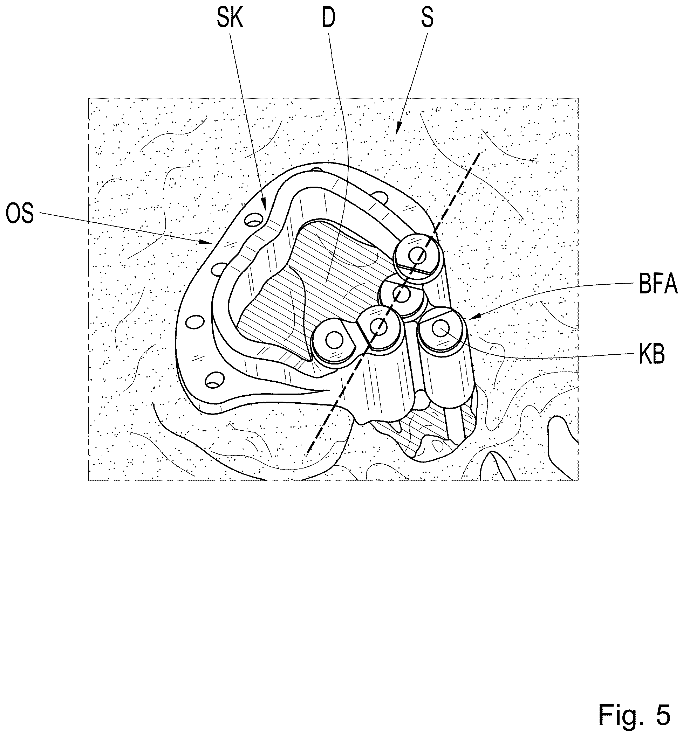

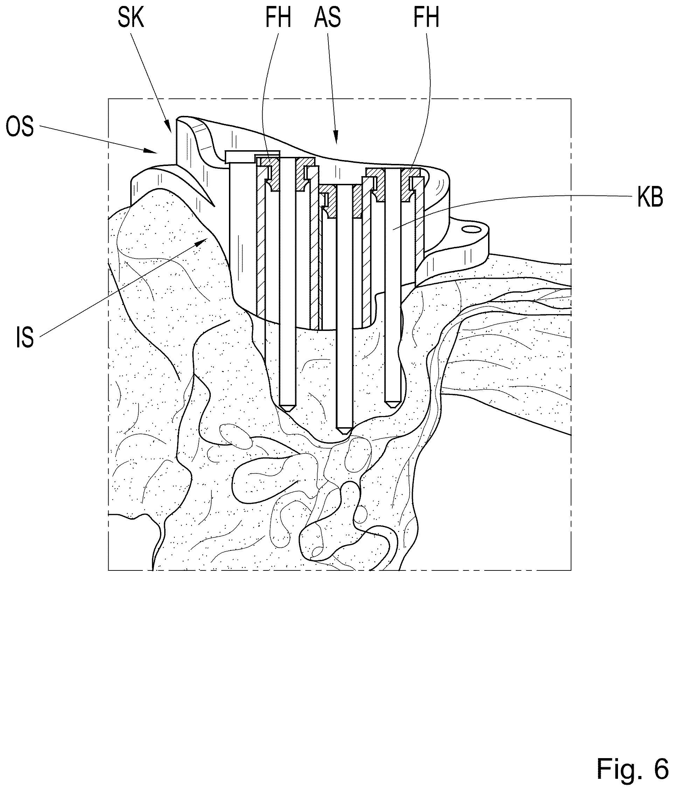

Weitere, zu entfernende Bereiche der Knochenabschnitte KA1 und KA2 werden bei der Operationsschablone OS zudem über die Bohrkanäle BK der Bohrführungsabschnitte BFA definiert, wobei der jeweilige Bohrkanal BK der Hindurchführung eines Knochenbohrers KB zum Entfernen je eines Knochenteils dient. Der Knochenbohrer KB ist hierbei in den Darstellungen in Fig. 5 und Fig. 6 jeweils angedeutet. In Fig. 6 sind dabei zudem die auf der Außenseite AS in den Schablonenkörper SK eingebrachten Führungshülsen FH zu erkennen, wobei die Innendurchmesser, die über die Führungsbereiche FB der Führungshülsen FH definiert sind, an einen Außendurchmesser des Knochenbohrers KB angenähert sind. Hierdurch wird eine Führung des Knochenbohrers KB in dem jeweiligen Bohrführungsabschnitt BFA realisiert.

Mithilfe der Anschlagsflächen ASF und der unterschiedlichen Längen der Bohrkanäle BK werden auf der Innenseite IS unterschiedliche Bohrtiefen des Knochenbohrers KB definiert. Dies ist hierbei in Fig. 6 zu erkennen. Zur Realisierung der jeweiligen Bohrtiefe muss der operierende Chirurg das den Knochenbohrer KB führende Operationswerkzeug jeweils bis auf Anschlag an die jeweilige Anschlagsfläche ASF führen.

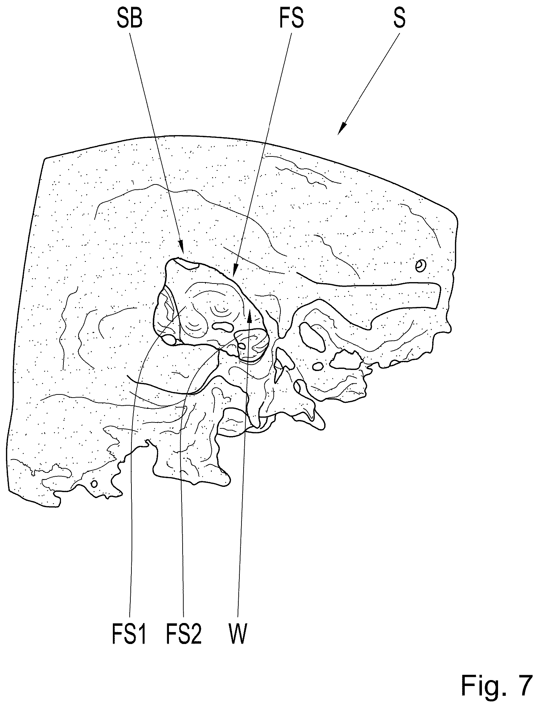

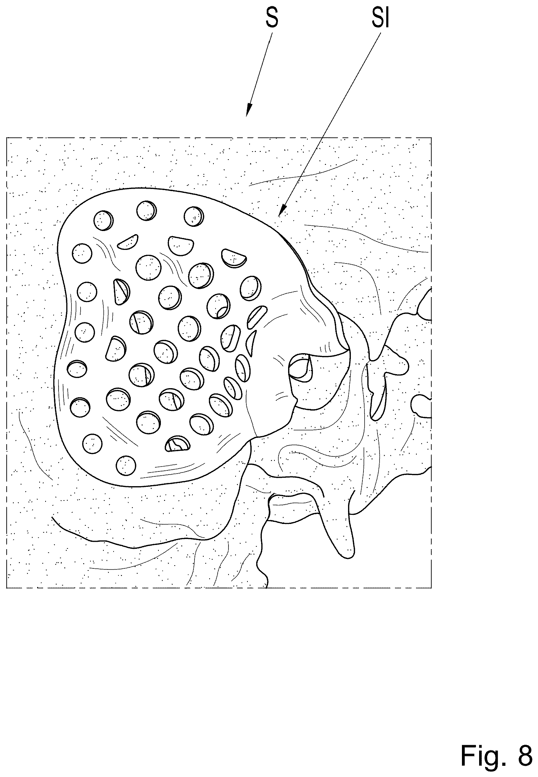

Ferner ist der Schädel S in Fig. 7 nach einem Entfernen der Knochenabschnitte KA1 und KA2 gezeigt, wodurch bei dem Schläfenbein SB eine Fehlstelle FS gebildet wurde. Dadurch, dass der Knochenabschnitt KA1 einen Abschnitt des Warzenteils und der Knochenabschnitt KA2 einen Abschnitt des Paukenteils bildet, weist die Fehlstelle FS einen Fehlstellenteil FS1 im Bereich des Warzenteils und einen Fehlstellenteil FS2 im Bereich des Paukenteils auf, wobei aufgrund Letzterem ein Abschnitt der Wand W entfernt worden ist. Zur Überdeckung dieser Fehlstelle FS wird an dem Schläfenbein SB ein Schläfenbeinimplantat SI befestigt, wie in Fig. 8 dargestellt ist. Dabei ist dieses Schläfenbeinimplantat SI gemeinsam mit der Operationsschablone OS auf Basis der erfassten Gestalt des Schädels S patientenspezifisch gestaltet worden.

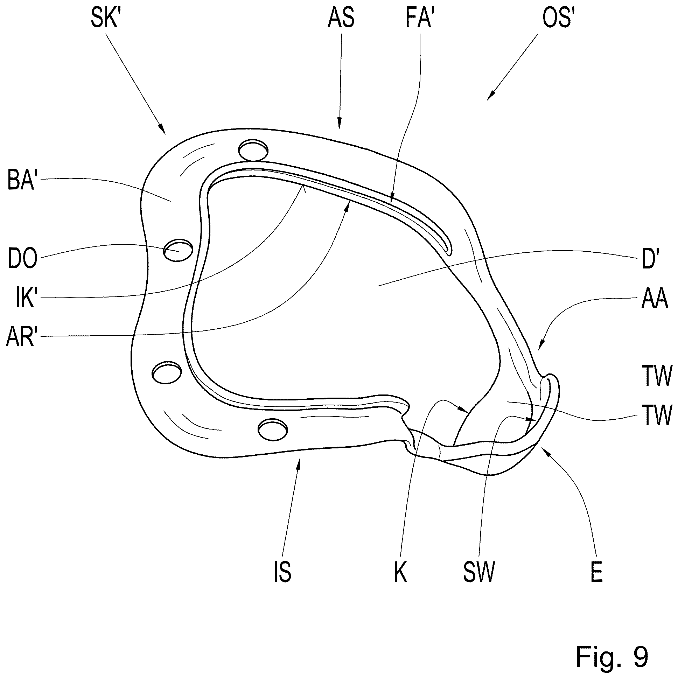

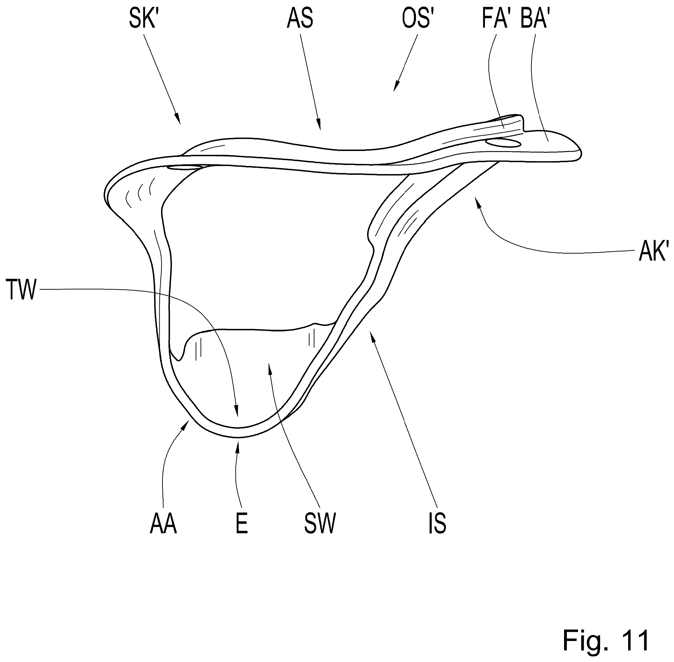

Des Weiteren gehen aus den Fig. 9 bis 11 unterschiedliche Ansichten einer Operationsschablone OS' entsprechend einer weiteren Ausgestaltungsmöglichkeit der Erfindung hervor. Diese Operationsschablone OS' ähnelt dabei der Variante nach Fig. 1 und ist ebenfalls für eine Anwendung im Bereich eines Schläfenbeins und hierbei insbesondere für die Durchführung einer radikalen Mastoidektomie vorgesehen.

Die Operationsschablone OS' ist erneut patientenspezifisch gestaltet und verfügt über einen Schablonenkörper SK', welcher im Unterschied zu der Variante nach Fig. 1 nun aber aus Metall und hierbei insbesondere aus Titan oder einer Titanlegierung besteht. Alternativ dazu kann die Operationsschablone OS' aus Kunststoff gefertigt sein. Der Schablonenkörper SK' ist auf einer Innenseite IS zum Auflegen auf das Schläfenbein gestaltet und - wie insbesondere in Fig. 9 zu erkennen ist - mit einem Durchbruch D' versehen, welcher den Schablonenkörper SK' von der Innenseite IS zu einer entgegengesetzt liegenden Außenseite AS durchsetzt. Der Schablonenkörper SK' ist ferner auf der Innenseite IS abschnittsweise mit einer Auflagekontur AK' versehen, welche im Rahmen der patientenspezifischen Gestaltung der Operationsschablone OS' zumindest annähernd als Gegenstück zu einer Oberflächenkontur des zugehörigen Schläfenbeins im aufzulegenden Bereich gestaltet ist.

Im Schablonenkörper SK' ist der Durchbruch D' dabei inselartig ausgebildet und dazu umlaufend mit einem Außenrand AR' umgrenzt, welcher abschnittsweise durch eine Innenkontur IK' eines Führungsabschnitts FA' sowie abschnittsweise durch eine Kante K eines Abdeckabschnitt AA definiert ist. Im Bereich des Führungsabschnitts FA' ist der Schablonenkörper SK' zudem mit einem Befestigungsabschnitt BA' versehen, welcher den Führungsabschnitt FA' peripher umgibt und der Durchgangsöffnungen DO aufweist. In analoger Weise zu der Variante nach Fig. 1 sind diese Durchgangsöffnungen DO dabei für das Hindurchführen von Knochenschrauben vorgesehen, über welche die Operationsschablone OS' in einer vorgesehenen Lage an dem Schläfenbein befestigt werden kann. Dazu verlaufen die Durchgangsöffnungen DO zwischen der Innenseite IS und der Außenseite AS. Zudem steht der Führungsabschnitt FA' gegenüber dem Befestigungsabschnitt BA' zu der Außenseite AS hin vor.

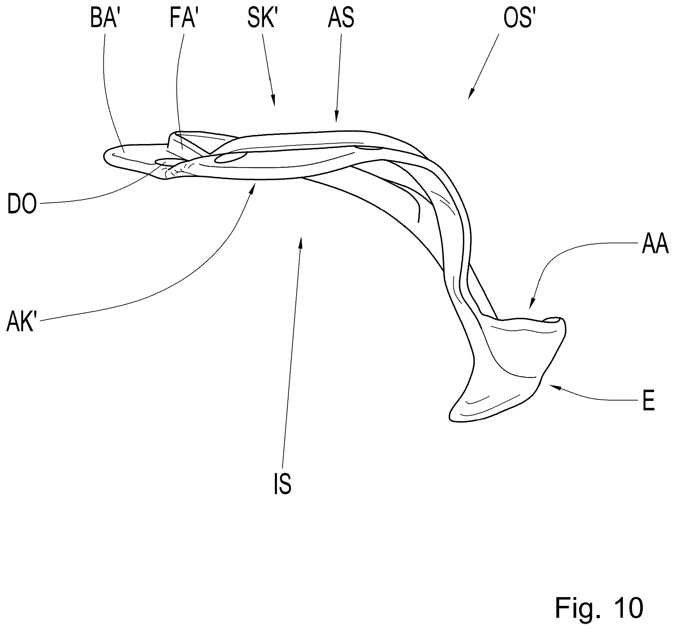

Wie insbesondere anhand von Fig. 10 erkennbar ist, ist der Abdeckabschnitt AA an einem Ende E des Schablonenkörpers SK' ausgestaltet, mit welchem Ende E der Schablonenkörper SK' gegenüber dem Befestigungsabschnitt BA' zu der Innenseite IS verkippt ist. Dabei ist der Abdeckabschnitt AA zu der Innenseite IS hin schaufelartig gewölbt ausgestaltet und bildet mit dieser Wölbung eine Trennwand TW sowie eine Seitenwand SW. Über die Trennwand TW ist angrenzend zu dem Durchbruch D' eine lokale Abteilung zwischen Innenseite IS und Außenseite AS vorgenommen. Die Seitenwand SW umgibt die Trennwand TW und steht dabei gegenüber der Trennwand TW zu der Außenseite AS des Schablonenkörpers SK' hin vor.

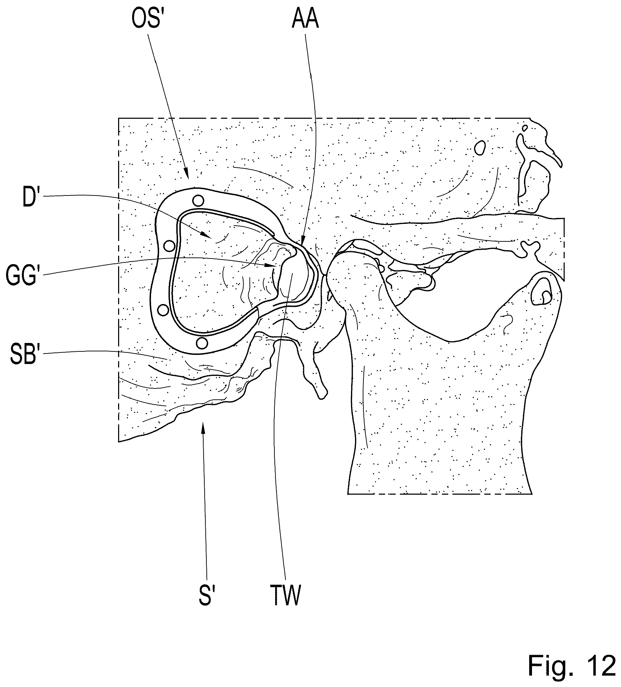

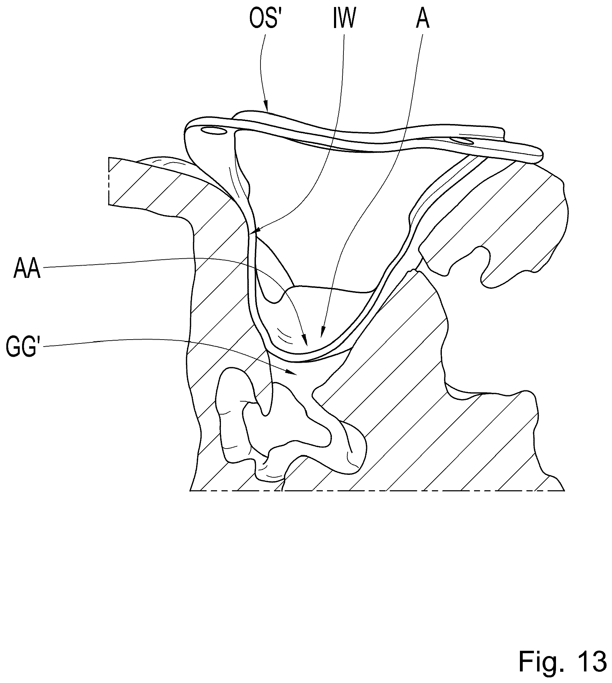

Fig. 12 und Fig. 13 zeigen die Operationsschablone OS' nach Fig. 9 bis Fig. 11 nun in einem Zustand, in welchem die spezifisch hierfür gestaltete Operationsschablone OS' auf ein Schläfenbein SB' eines Schädels S' aufgelegt ist. Wie insbesondere in Fig. 12 zu erkennen ist, wird dabei durch die Trennwand TW des Abdeckabschnitts AA ein Gehörgang GG' vollständig überdeckt, wobei die Operationsschablone OS' dabei zudem mit dem Abdeckabschnitt AA in den Gehörgang GG' eingeführt ist. Dies ist dabei insbesondere anhand von Fig. 13 ersichtlich, wobei zudem zu erkennen ist, dass der Abdeckabschnitt AA in dem befestigten Zustand Operationsschablone OS' zum Teil an einer Innenwand IW des Gehörganges GG' anliegt.

Durch den Durchbruch D' der befestigten Operationsschablone OS' wird dabei an dem Schläfenbein SB' der zu entfernende Knochenteil identifiziert. Über den Abdeckabschnitt AA wird dabei zudem wirksam ein Verletzen des Gehörgangs GG' verhindert, indem die Trennwand TW ein Abrutschen in den Gehörgang GG' unterbindet sowie die Seitenwand SW die beizubehaltende Innenwand IW des Gehörgangs GG' schützt.

Darüber hinaus wird durch die Trennwand TW eine Auflage A gebildet, auf welcher der die Operation durchführende Chirurg das OP-Werkzeug auflegen kann und durch die eine Resektionstiefe definiert ist. Dadurch kann sich der operierende Chirurg beispielsweise mithilfe eines Knochenfräsers ausgehend von der Auflage A seitlich in den zu entfernenden Knochen hineinarbeiten. Hierfür ist der Schablonenkörper SK' der Operationsschablone OS' dann auch im Bereich des verkippten Endes E nicht mit dem Führungsabschnitt FA' versehen, an welchem der operierende Chirurg ansonsten mit dem OP-Werkzeug entlangfahren kann.

Mittels der erfindungsgemäßen Ausgestaltungen kann jeweils eine Operationsschablone geschaffen werden, mittels welcher ein Chirurg bei der Durchführung einer Operation im Bereich des Schläfenbeins, bevorzugt einer radikalen Mastoidektomie, unterstützt werden kann.

### Bezugszeichenliste

- OS, OS': Operationsschablone
- SK, SK': Schablonenkörper
- IS: Innenseite
- D, D': Durchbruch
- AS: Außenseite
- AR, AR': Außenrand
- IK, IK': Innenkontur
- FA, FA': Führungsabschnitt
- BA, BA': Befestigungsabschnitt
- DÖ: Durchgangsöffnungen
- BFA: Bohrführungsabschnitte
- BK: Bohrkanal
- FH: Führungshülsen
- FB: Führungsbereich
- ASF: Anschlagfläche
- AK, AK': Auflagekontur
- SB, SB': Schläfenbein
- S, S': Schädel
- KA1, KA2: Knochenabschnitte
- GG, GG': Gehörgang
- W: Wand
- KB: Knochenbohrer
- FS: Fehlstelle
- FS1, FS2: Fehlstellenteile
- SI: Schläfenbeinimplantat
- K: Kante
- AA: Abdeckabschnitt
- E: Ende
- TW: Trennwand
- SW: Seitenwand
- IW: Innenwand
- A: Auflage

## Patentansprüche

1. Operationsschablone (OS; OS') zur Anwendung im Bereich eines Schläfenbeins (SB; SB'), insbesondere im Rahmen einer Durchführung einer radikalen Mastoidektomie, umfassend einen Schablonenkörper (SK; SK') mit einer Außenseite (AS) und einer Innenseite (IS), auf welcher der Schablonenkörper (SK; SK') schläfenbeinseitig aufzulegen ist, **dadurch gekennzeichnet, dass** der Schablonenkörper (SK; SK') mit einem von der Außenseite (AS) zu der Innenseite (IS) verlaufenden Durchbruch (D; D') durchsetzt ist, und dass der Durchbruch (D; D') mit einem Außenrand (AR; AR') definiert ist, welcher einer Außenkontur eines zu entfernenden Knochenteils des Schläfenbeins (SB; SB') entspricht.

2. Operationsschablone (OS; OS') nach Anspruch 1, **dadurch gekennzeichnet, dass** der Außenrand (AR; AR') zumindest abschnittsweise durch eine Innenkontur (IK; IK') eines Führungsabschnitts (FA; FA') der Operationsschablone (OS; OS') gebildet ist.

3. Operationsschablone (OS; OS') nach Anspruch 1 oder 2, **dadurch gekennzeichnet, dass** der Schablonenkörper (SK; SK') mit einem Befestigungsabschnitt (BA; BA') versehen ist, an welchem von der Außenseite (AS) zu der Innenseite (IS) verlaufende Durchgangsöffnungen (DO) ausgestaltet sind, wobei die Durchgangsöffnungen (DO) zur Hindurchführung je eines Befestigungsmittels für eine schläfenbeinseitige Befestigung der Operationsschablone (OS; OS') eingerichtet sind, bevorzugt für eine Befestigung am Warzenteil des Schläfenbeins (SB; SB').

4. Operationsschablone (OS; OS') nach den Ansprüchen 2 und 3, **dadurch gekennzeichnet, dass** der Befestigungsabschnitt (BA; BA') den Führungsabschnitt (FA; FA') zumindest abschnittsweise peripher umgibt, wobei der Führungsabschnitt (FA; FA') gegenüber dem Befestigungsabschnitt (BA; BA') zu der Außenseite (AS) hin vorstehend ausgestaltet ist, oder der Befestigungsabschnitt (BA) stufenlos in den Führungsabschnitt (FA) übergeht.

5. Operationsschablone (OS') nach wenigstens einem der vorhergehenden Ansprüche, **dadurch gekennzeichnet, dass** der Schablonenkörper (SK') einen Abdeckabschnitt (AA) aufweist, welcher eine Trennwand (TW) bildet, wobei die Trennwand (TW) im Bereich des Abdeckabschnitts (AA) die Außenseite (AS) und die Innenseite (IS) voneinander abteilt und für ein zumindest überwiegendes Überdecken eines Gehörgangs (GG') durch die Operationsschablone (OS') vorgesehen ist.

6. Operationsschablone (OS') nach Anspruch 5, **dadurch gekennzeichnet, dass** der Abdeckabschnitt (AA) zu der Innenseite (IS) hin gewölbt gestaltet ist und einer Einführung in den Gehörgang (GG') dient, wobei der Abdeckabschnitt (AA) dabei neben der Trennwand (TW) zudem eine Seitenwand (SW) bildet, die gegenüber der Trennwand (TW) zu der Außenseite (AS) hin vorsteht.

7. Operationsschablone (OS') nach Anspruch 5 oder 6, **dadurch gekennzeichnet, dass** der Abdeckabschnitt (AA) bei dem Schablonenkörper (SK') an einem Ende (E) vorgesehen ist, mit welchem der Schablonenkörper (SK') zu der Innenseite (IS) hin verkippt ist, wobei der Abdeckabschnitt (AA) einen Abschnitt des Außenrandes (AR') des Durchbruchs (D') durch eine Kante (K) der Trennwand (TW) definiert, wobei der Abdeckabschnitt (AA) vorzugsweise eine Auflage (A) bildet, welche einem Auflegen eines zum Entfernen des Knochenteils des Schläfenbeins (SB') zu verwendenden Operationswerkzeugs dient und eine Resektionstiefe definiert.

8. Operationsschablone (OS') nach Anspruch 2 und nach Anspruch 7, **dadurch gekennzeichnet, dass** der Außenrand (AR') des Durchbruchs (D') abseits des verkippten Endes (E) durch die Innenkontur (IK') des Führungsabschnitts (FA') gebildet ist.

9. Operationsschablone (OS) nach wenigstens einem der vorhergehenden Ansprüche, **dadurch gekennzeichnet, dass** an dem Schablonenkörper (SK) zudem mindestens ein Bohrführungsabschnitt (BFA) ausgestaltet ist, welcher zumindest einen von der Außenseite (AS) zu der Innenseite (IS) verlaufenden Bohrkanal (BK) bildet und der jeweils einer Führung eines Knochenbohrers (KB) zum Entfernen je eines Knochenteils des Schläfenbeins (SB) dient.

10. Operationsschablone (OS) nach Anspruch 9, **dadurch gekennzeichnet, dass** der mindestens eine Bohrführungsabschnitt (BFA) zumindest an einem an der Außenseite (AS) liegenden Ende mit jeweils einem Führungsbereich (FB) versehen ist, in welchem ein Innendurchmesser des Bohrkanals (BK) an einen Außendurchmesser des Knochenbohrers (KB) angenähert ist.

11. Operationsschablone (OS) nach Anspruch 10, **dadurch gekennzeichnet, dass** der jeweils eine Führungsbereich (FB) durch eine Führungshülse (FH) gebildet ist, welche an dem mindestens einen Bohrführungsabschnitt (BFA) in den Schablonenkörper (SK) eingebracht ist.

12. Operationsschablone (OS) nach wenigstens einem der Ansprüche 9 bis 11, **dadurch gekennzeichnet, dass** an dem mindestens einen Bohrführungsabschnitt (BFA) bündig an einer außenseitigen Mündung des Bohrkanals (BK) eine Anschlagsfläche (ASF) ausgestaltet ist, welche einen Anschlag für ein den Knochenbohrer (KB) führendes Operationswerkzeug bildet, wobei eine Länge des jeweiligen Bohrkanals (BK) entsprechend einer Bohrtiefe gestaltet ist, welche über den Knochenbohrer (KB) auf der Innenseite (IS) bei Anschlag des Operationswerkzeug an der jeweils einen Anschlagsfläche (ASF) darzustellen ist.

13. Operationsschablone (OS) nach wenigstens einem der Ansprüche 9 bis 12, **dadurch gekennzeichnet, dass** mehrere Bohrführungsabschnitte (BFA) ausgestaltet sind.

14. Operationsschablone (OS) nach Anspruch 2 und wenigstens einem der Ansprüche 9 bis 13, **dadurch gekennzeichnet, dass** der mindestens ein Bohrführungsabschnitt (BFA) an dem Führungsabschnitt (FA) angrenzend zu der Innenkontur (IK) ausgestaltet ist.

15. Operationsschablone (OS; OS') nach wenigstens einem der vorhergehenden Ansprüche, **dadurch gekennzeichnet, dass** der Schablonenkörper (SK; SK') patientenspezifisch ausgeführt ist.

16. Operationsschablone (OS; OS') nach Anspruch 15, **dadurch gekennzeichnet, dass** der Schablonenkörper (SK; SK') auf der Innenseite (IS) mit einer Auflagekontur (AK; AK') ausgeführt ist, welche zumindest annähernd als Gegenstück zu einer Oberflächenkontur des Schläfenbeins (SB; SB') im aufzulegenden Bereich gestaltet ist.

17. Verfahren zur Gestaltung und/oder Herstellung einer Operationsschablone (OS; OS') für eine Anwendung im Bereich eines Schläfenbeins (SB; SB'), wobei ein Schablonenkörper (SK; SK') mit einer Außenseite (AS) und einer Innenseite (IS) gestaltet wird, auf welcher der Schablonenkörper (SK; SK') schläfenbeinseitig aufzulegen ist, wobei bei dem Schablonenkörper (SK; SK') ein von der Außenseite (AS) zu der Innenseite (IS) verlaufender Durchbruch (D; D') vorgesehen wird, und wobei bei dem Durchbruch (D; D') ein Außenrand (AR; AR') definiert wird, welcher einer Außenkontur eines zu entfernenden Knochenteils des Schläfenbeins (SB; SB') entspricht.

18. Verfahren nach Anspruch 17, **dadurch gekennzeichnet, dass** der Schablonenkörper (SK; SK') im Weiteren nach wenigstens einem der Ansprüche 2 bis 16 gestaltet wird.

19. Verfahren nach Anspruch 17 oder 18, **dadurch gekennzeichnet, dass** gemeinsam mit der Operationsschablone (OS; OS') zudem ein Schläfenbeinimplantat (SI) geplant und/oder hergestellt wird.
